# EUROPEAN PATENT APPLICATION

(11) **EP 4 282 398 A1**
(43) Date of publication of application: **29.11.2023**
(21) Application number: 23174257.8
(22) Date of filing: 19.05.2023
(51) Int. Cl.: A61K 8/44, A61K 8/67, A61Q 5/02, A61Q 5/12, A61Q 7/00

(54) **HAIR CARE COMPOSITION**

(30) Priority: 23.05.2022 IT 202200010682
(71) Applicant: Sanders S.r.l., 20124 Milano (IT)
(72) Inventor: ANGELINI, Matteo, London (GB)
(74) Representative: Rapisardi, Mariacristina

(57) **Abstract**

A hair care composition characterised in that it comprises *Pisum sativum* seed extracts as anti-hair loss agents and densifiers for hair growth in synergistic association with betaine, niacinamide, *Cucurbita pepo* seed and/or fruits extracts, eucalyptus extract and birch leaf extracts for use in the treatment of skin alterations of the scalp and for use in the promotion of hair shaft regrowth.

## Description

The present invention relates to a hair care composition.

The present invention relates to the cosmetic and phytocosmetic sector and, more specifically, to the sector of natural compositions for the treatment of hair and scalp, in particular in cases of imperfections related to skin alterations of the scalp, also in the presence of itchy symptoms, in order to improve and promote hair regrowth.

### Known technique

The scalp is the area of the skin where the hair resides. As a biological material subject to alterations, abnormalities, disorders or actual diseases, the scalp has, like the entire integumentary apparatus, the task of providing protection and thermoregulation. Hair also amplifies protective and thermoregulatory actions, and plays an important role in social and sexual communication.

The health of the scalp is closely related to the well-being and growth of hair; in fact, it is where the hair follicles, the organs that produce hair, reside.

The problem of hair loss is widespread and affects many people, both men and women, and also affects their quality of life and interpersonal relationships.

When faced with a balding or thinning problem, the first thing to do is to observe the scalp and understand its state of health.

The most common scalp abnormalities occur through itching, trichodynia, flaking, dandruff and seborrhea.

Dandruff occurs due to an accelerated turnover of epidermal cells of the scalp, which, migrating faster, are unable to mature before detachment; this results in excessive desquamation of the superficial horny layer of the skin epidermis.

Dandruff is widespread and affects more than 40% of the male and female population, mostly young people.

Dandruff causes skin irritation often accompanied by itching, bacterial proliferation and possible degeneration into dermatitis and dermatosis.

Seborrhea, like dandruff, is also an important factor predisposing to thinning hair. In fact, an excess of sebum, due to hypertrophy of the sebaceous glands of the scalp hyper stimulated by the hormone DHT (dihydrotestosterone), considered one of the main causes of hair loss, tends to change the chemical-physical characteristics of the peri- and intrafollicular environment, altering the biochemical balances responsible for proper hair growth and consequently miniaturisation of regrowth and premature hair loss.

To remedy these conditions, it would be good to have hair care compositions and products that can improve quality of life by eliminating skin alterations that cause itchy symptoms and that can promote hair regrowth by improving hair strength and aesthetics.

In fact, the task of the present invention is to provide a hair care composition to solve the drawbacks described in the known technique.

In the context of this technical task, one aim of the present invention is to provide a hair care composition that can act simultaneously and specifically on the scalp, at the intrafollicular level and on the hair shaft.

Another purpose of the present invention is to provide a hair care composition that is predominantly of natural origin.

A further purpose of the present invention is to provide a hair care composition characterised by the combination of natural, non-toxic compounds and free of harsh chemical surfactants.

These and other purposes of the present invention are achieved by means of a hair care composition characterised in that it comprises *Pisum sativum* seed extracts as anti-hair loss agents and densifiers for hair growth in synergistic association with betaine, niacinamide, *Cucurbita pepo* seed and/or fruits extracts, eucalyptus extract and birch leaf extracts for use in the treatment of skin alterations of the scalp and for use in the promotion of hair shaft regrowth. Other salient aspects of the invention are set out in the dependent claims below.

The figures below refer to the experimental tests carried out to highlight the synergistic effect associated with the combination of the components of the hair care composition:
- Figure 1 shows a graph of the initial condition at time 0 (T0) of the persons involved in the experimental tests with reference to the Pull Test;
- Figure 2 shows a graph of the initial condition at time 0 (T0) of the persons involved in the experimental tests with reference to the comb test;
- Figure 3 shows a graph of the initial condition at time 0 (T0) of the scalp of the people involved in the experimental tests;
- Figure 4 shows a graph of the initial symptomatology at time 0 (T0) presented by the persons involved in the experimental tests;
- Figure 5 shows a graph of Pull test and comb test results evaluated 5 weeks (T5) after the start of treatment in comparison to T0 in each group of testers treated with a formulation called FORMi, with i from 1 to 8;
- Figure 6 shows a graph of normalization of the scalp and symptomatology assessed 5 weeks (T5) after the start of treatment compared to T0 in each group of testers treated with a formulation called FORMi, with i from 1 to 8;
- Figure 7 shows a graph of the percentage of thickening of the hair shaft at time T5 compared to T0 after the start of treatment in each group of testers treated with a formulation called FORMi, with i from 1 to 8;
- Figure 8 shows a graph of the perception of pleasure and well-being derived from the treatment used at T5 compared to T0 in each group of testers treated with a formulation called FORMi, with i from 1 to 8;
- Figure 9 shows a summary and comparative graph of the results obtained at time T5 with reference to the different variables tested (comb test, pull test, symptomatology, normalization of the scalp, thickening and perception of pleasure and personal well-being) compared to T0 in each group of testers treated with a formulation called FORMi, with i from 1 to 8.

The hair care composition according to the present invention advantageously includes *Pisum Sativum* seed extracts as strong anti-hair loss and hair growth densifying agents.

In particular, *Pisum Sativum* is synergistically combined with betaine, niacinamide, curcubita pepo seed and/or fruits extracts, eucalyptus extract and betula leaf extracts for use in the treatment of scalp disorders and for use in promoting hair shaft regrowth.

The amount of *Pisum Sativum* extract present in the composition varies from 0.5 to 2% of the total weight, the amount of betaine present in the composition varies from 0.5% to 3% of the total weight, the amount of niacinamide present varies from 0.5% to 2% of the total weight, the amount of *Curcubita pepo* seed and/or fruits extracts present varies from 0.5 to 2% of the total weight, the amount of eucalyptus extract varies from 0.5% to 3% of the total weight, and the amount of betula leaf extract varies from 0.05% to 0.20% of the total weight.

The composition in particular is characterised by the combination of natural, non-toxic compounds and is free of chemical surfactants, making it particularly suitable for treating blemishes such as dandruff and seborrhea of the scalp.

The composition also includes hydrolyzed wheat proteins in combination with betaine and niacinamide for use in restoring the correct hydrolipid balance of the scalp.

In addition to the above-mentioned components, the composition also includes caffeine, panthenol in synergistic association with *Pisum Sativum* seed extracts for use in the treatment of hair loss.

In addition to the synergistic natural mixture of betaine, niacinamide, curcubita pepo seed and/or fruits extracts, eucalyptus extract, betula leaf extracts, hydrolysed wheat protein, caffeine, panthenol and *Pisum Sativum* seed extracts, the hair care composition according to the invention includes further components, additives, excipients, fragrances and colouring agents well known to experts in the field.

For example, the composition includes propanediol and glycerine as wetting agents, heptylglucoside as surfactant agent, tetrasodium glutamate diacetate as plant-based chelating agent, cetrimonium chloride as antistatic agent and citric acid as PH-regulating agent.

The extraction method of the extracts in the claimed composition is based on a hydro-glycol solvent, specifically propanediol.

Solvents that may further be present in the composition according to the invention are water, denatured alcohol, and other agents to promote solubilization of the various components known in the industry.

The composition according to the invention is in a form suitable for topical application to the hair and scalp, in particular in the form of shampoos, conditioners, gels, creams, masks and lotions.

The invention will now be illustrated with reference to formulation examples and in particular with reference to the phytotherapeutic characteristics of each individual component.

### EXAMPLE 1

In a particularly preferred embodiment, the composition according to the invention has the following formulation.

| **Ingredients** | **% by Total weight** |
|---|---|
| Water | 64,2845 |
| Denatured alcohol | 26,190 |
| Propanediol | 2,75 |
| Betaine | 2,00 |
| Glycerine | 1,00 |
| Niacinamide | 1,00 |
| Hydrolysed wheat protein | 1,00 |
| Caffeine | 0,50 |
| Panthenol | 0,50 |
| Heptylglucoside | 0,20 |
| Extract of Eucalyptus Globulus | 0,20 |
| Tetrasodium glutamate diacetate | 0,10 |
| Curcubita pepo seed extracts | 0,10 |
| Pisum Sativum Seed Extracts | 0,10 |
| Betula leaf extracts | 0,050 |
| Cetrimonium chloride | 0,025 |
| Citric acid | 0,0005 |

The composition must be used topically to provide a skin correction, as scalp disorders are often the cause or concomitant cause of thinning and often associated with uncomfortable and incessant itching causing local scratching irritation.

The synergic action of betaine, niacinamide, cucurbita pepo, eucalyptus and betula is effective in treating dermatitis, flaking and hyper seborrhea, itching and trichodynia. Cucurbita pepo also has a local anti- androgenic effect.

A great importance for the well-being of skin and hair is given to the balance of the hydrolipidic layer, which is indeed the normal natural skin barrier.

To rebalance and maintain the correct hydrolipid balance of the skin, betaine and niacinamide are used in synergy with hydrolyzed wheat proteins.

Hair growth occurs during the anagen phase, and is promoted by caffeine in synergy with panthenol and *Pisum Sativum.* The latter in particular also has an anti-hair loss effect.

An important role in shaft thickness is played by the peptide of hydrolyzed wheat protein and *Pisum Sativum,* a valuable and complete source of available amino acids.

Finally, the aesthetics of the hair, in the sense of volume, elasticity, strength and shine, is promoted by the revitalising and moisturising action of the many synergetic active ingredients, of which panthenol remains the mainstay.

Specifically, we will outline the properties of all the active ingredients contained in the composition according to the present invention.

### BETAINE

Thin crystals extracted from sugar beet with important moisturising properties. It is used in skin and hair care to counteract dehydration and environmental stress.

The particular structure of betaine allows it to act as an osmolyte, i.e. it is capable of attracting water to itself and helps keep the concentration of cellular water in balance. If the scalp is dehydrated and prone to cracking, itching, or the hair shaft dull and dry, the osmosis created by betaine allows the flow of water to penetrate into that area to recreate a new balance. This function of betaine is particularly crucial for dry skin and hair exposed to UV radiation and thus to photo-ageing. The hair is strengthened, more elastic and more voluminous as a result.

In fact, the composition according to the present invention has an excellent concentration of betaine, so that it acts in a specific and targeted manner in protecting and reinforcing the normal hydrolipidic film that is indispensable as protection for the skin, which is firmer and more elastic. It also reduces chemical and mechanical irritation caused by cosmetic practices or environmental stress and has an anti-inflammatory effect.

The benefits of betaine in skin and hair care are thus expressed in a moisturizing, anti-inflammatory, anti-ageing and soothing effect.

Considering the biocompatibility of the active ingredients used, it is important to emphasize that in addition to its beneficial effects, betaine occurs naturally in our bodies, so it is a non-allergenic and safe ingredient.

### NIACINAMIDE

Niacinamide is the physiologically active form of niacin or vitamin B3, an odorless white crystalline powder that is stable when exposed to heat, light, air and alkalis.

Niacinamide enables us to act on skin and hair through its antimicrobial, vaso-activating, photo-protective and sebum-static effects. Its action on hyper seborrhea is implemented through the regulation of skin lipids. It is also a well-tolerated and safe substance with an important bioavailability.

Vitamin B3 was also chosen for its anti-ageing effect, as it inhibits the degradation of collagen, a very important element of the dermis of the skin and induces the healing of wounds and small lesions on the scalp.

Treatment with vitamin B3 has a positive effect on the stratum corneum, improving hydration, in combination with betaine it ensures the correct balance of the skin and stems while also enhancing aesthetics.

Desquamation, dandruff and seborrhea result in an alteration of the skin microbiota and hydrolipidic barrier that makes the scalp more vulnerable to infection. A good concentration of B3 allows anti-inflammatory action where the scalp is altered and maintains the integrity of the epidermal barrier by protecting the scalp from microbiological infections responsible for many skin imperfections and associated symptomatology.

At the shaft level, vitamin B3 stimulates keratin synthesis, increasing its thickness, strength and shine.

### HYDROLYZED WHEAT PROTEIN

In cosmetics, wheat proteins are used as peptides for skin and hair care. Peptides (medium molecular weight proteins) are very important in all living organisms, of which they are one of the most important components. Wheat proteins in particular, in addition to their protein component, provide hydration as they draw water into the deep layers of the epidermis.

Wheat Protein is ideal for dull, dry, brittle and lifeless hair. It has been proven in clinical tests that Wheat Protein aids the reconstruction of hair fibres and increases their overall strength and resistance. Due to its properties and film-forming effect, hair becomes softer, fuller and more vital. The scalp becomes smoother and more elastic.

Together with Betaine and Niacinamide, it aids local hydration and, thanks to the excellent penetration of these proteins into the tissue, enables deep tissue protection and restructuring involving the follicular environment.

Wheat proteins are recognised as a green chemical solution.

### CAFFEINE

Caffeine has long been used in cosmetics for its lipolytic properties. By increasing the concentration of cellular AMPc, it activates lipase, which causes the hydrolysis of triglycerides in adipocytes. The increase in AMPc is also responsible for lengthening the Anagen phase with an anti-fall effect.

Caffeine is a purine alkaloid found in many nuts, leaves, coffee, tea, cocoa and other plants and was recognised in 1958 by the US Food and Drug Administration (FDA) as safe.

In biological terms, caffeine has many effects, the best known of which are anti-oxidant, anti-ageing, vaso-activating and anti-inflammatory. Used topically, it helps reduce redness and swelling and protects against UVB rays and photo-ageing.

### PANTENOL

Panthenol (D-pantothenyl alcohol), or Pro-Vitamin B5, is rapidly converted and utilized in the form of pantothenic acid (Vitamin B5) after penetrating the skin. Once applied to the skin, panthenol penetrates deep into the epidermis.

As a component of coenzyme A, pantothenic acid has an important metabolic function; its main properties are:
- increased mitotic activity and thus cell regeneration, greater ease of healing.
- anti-inflammatory
- moisturizing, restores and maintains the skin's natural moisture content
- diminishes the effects of the sun's rays

Panthenol deficiency has been seen to cause dermatitis, depigmentation, and desquamation of the epidermis.

Panthenol also penetrates deep into the hair shaft to promote hydration, increase its strength and thickness and make the hair appear silkier.

### EUCALYPTUS GLOBULUS

Antibacterial, it purifies and tones oily skin and skin problems also related to thinning and seborrheic alopecia.

Antioxidant and stimulating, but also antifungal, making it an exceptional remedy for skin dermatitis associated with itchy symptoms.

### CUCURBITA PEPO

Cucurbita Pepo seed and/or fruits extracts provides a re-lipidification of the skin barrier, but without greasing. On the contrary, it has an immediately visible mattifying effect on usually oily and greasy skin. Its sebum-regulating action is excellent on oily skin and seborrheic alopecia. It also has an anti-androgenic action on the 5 alpha reductase enzyme, making it useful in the treatment of alopecias, including androgenetic ones.

### PISUM SATIVUM

The peptides it contains are gaining credence as skin and hair care. In fact, the peptides contained in *Pisum Sativum* are a complete source of essential amino acids, particularly lysine, which is an essential building block of human cells. This unique balance of nutrients gives this nourishing peptide important volumising, anti-ageing, cell regeneration, antioxidant, conditioning and hair-strengthening properties.

When applied topically to the hair, pea peptide is believed to help stimulate and strengthen new hair growth by preventing premature hair loss. Many data in the literature have demonstrated the effectiveness of *Pisum Sativum* as a strong anti-hair loss and hair regrowth enhancer.

### BETULA

Birch leaf extract is known for its many virtues. It acts as an astringent and skin purifier. Birch leaf extract also has soothing properties that leave a feeling of well-being and comfort on the scalp.

Main properties of birch leaf extract:
- astringent
- soother
- Invigorating

The special synergic combination of betaine, niacinamide, cucurbita pepo, eucalyptus, betula, hydrolysed wheat protein, caffeine and pisum sativum ensures normalization of the scalp and enables correction of scalp imperfections (e.g. dandruff, seborrhea, redness, etc.) that are often conducive to hair loss.

### STUDY EVALUATING THE SYNERGISTIC ACTION OF THE ACTIVE INGREDIENTS IN THE COMPOSITION

The following will describe the study carried out by the inventors of the present invention in order to evaluate the specific synergistic action of the main actives constituting the claimed composition.

The aim of the study is to demonstrate that the synergistic action of the main active ingredients of the claimed hair care composition leads to trichological results superior to those achieved by the action of individual ingredients.

The different formulations for topical use were administered over a 5-week period in the treatment of people presenting trichological problems and associated symptoms such as thinning hair, hair loss, fragile regrowth, flaking, hyper seborrhea, itching and trichodynia. Selected testers used specific double-blind formulations differentiated into:
- 5 testers used a formulation containing only Betaine + Niacinamide
- 5 testers used a formulation containing only Hydrolyzed Wheat Protein
- 5 testers used a formulation containing only Caffeine
- 5 testers used a formulation containing only panthenol
- 5 testers used a formulation containing only Pisum Sativum
- 5 testers used a formulation containing only Cucurbita Pepo
- 5 testers used a complete formulation according to the present invention.
- 5 testers used a placebo formulation.

The study was carried out in a double-blind, case-controlled manner, and aimed to record the change in certain parameters following treatment:
- the reduction of hair loss (semeiological tests),
- density and diameter (microcamera and microscopy)
- scalp health (objective examination and microcamera)
- personal perception questionnaire.

Evaluations were carried out by means of a form filled out by the healthcare professional at time 0 (T0) and after 5 weeks (T5) of treatment in the 8 different formulations.

### MATERIALS AND METHODS

40 testers were recruited, divided into groups of five, who used the following 8 formulations topically according to the specific procedure described later on:
1. Betaine 2% (632302) + Niacinamide 1% + 26% alcohol + water for the remaining percentage (Form 1)
2. Hydrolized Wheat Protein 1% + 26% alcohol + water for the remaining percentage (Form 2)
3. Caffeine 0.5% + 26% alcohol + water for the remaining percentage (Form 3)
4. Panthenol 0.5% + 26% alcohol+ water for the remaining percentage (Form 4)
5. Pisum Sativum Seed Extract 0.1% + 26% alcohol + water for the remaining percentage (Form 5)
6. Cucurbita Pepo Fruit / Seed Extract 0.1% + 26% alcohol + water for the remaining percentage (Form 6)
7. Complete formulation of the composition according to the present invention: Betaine 2% (632302) + Niacinamide 1% +Hydrolized Wheat Protein 1% + Caffeine 0.5% + Panthenol 0.5% + Pisum Sativum Seed Extract 0.1% + Eucalyptus Globulus Leaf extract 0.2% + Cucurbita Pepo Fruit / Seed Extract 0.1% + Betula Alba Leaf Extract 0.05% + 26% alcohol + water for the remaining percentage (Form 7)
8. Placebo: 26% alcohol + water for the remaining percentage (Form 8, placebo).

Each tester was asked to massage 10 ml of the 8 formulations three times a week and hold them for at least 8 hours before performing a complete hygienic phase.

The biologist records the data of all 40 testers in the study from the beginning (T0) and describes their situation by filling in a declarative data table:
- positive or negative pull test,
- comb test in terms of the number of hairs fallen out,
- the presence of hyper seborrhea and/or flaking
- The presence of symptoms such as itching and/or trichodynia
- the average diameter of capillary hair shaft in micrometres,
- the perception of aesthetics and personal well-being using a scale from 0 to 10 where 0 indicates a situation of severe skin discomfort or very low aesthetic satisfaction and 10 maximum well-being and excellent aesthetic performance.

The trichological situation of in-depth instrumental examination (microcamera and microscope) is recorded with photographic documentation. The professional biologist, after an objective and instrumental examination at T0, repeats the tests and completes the questionnaire at T5, evaluating and reporting the results of all tests already performed at T0.

### INCLUSION AND EXCLUSION CRITERIA

40 people were selected, aged between 18 and 79 years, who presented some or all of the trichological discomforts under study: thinning- hyper seborrhea, desquamation, itching, trichodynia, hair loss, aesthetically unsatisfactory mass. Exclusion criteria for the purposes of the study were the presence of psychiatric pathologies in the volunteer, the presence of precancerous skin and systemic malignant conditions or serious systemic pathologies such as diabetes or cirrhosis, and finally being under treatment with topical or systemic pharmacological formulations.

### CONDUCT OF THE DOUBLE-BLIND STUDY WITH CONTROL GROUP

The 40 testers were blindly assigned one of 8 formulations, without them knowing the contents of the formulations, so that each formulation was tested by 5 people. All 40 testers followed the specific procedure describe above for the application of the formulations and the hygiene performed. The biologist recorded the results of the study at T1 and T5 and documented them. In addition to the visual and instrumental results by the biologist, the personal perception of the tester was recorded.

### STUDY SAMPLE

The study involved a total of 40 people aged between 18 and 78.

### At T0 there were the following initial conditions:

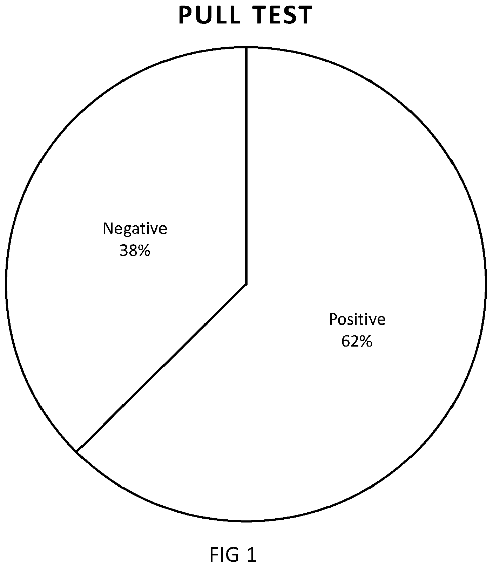

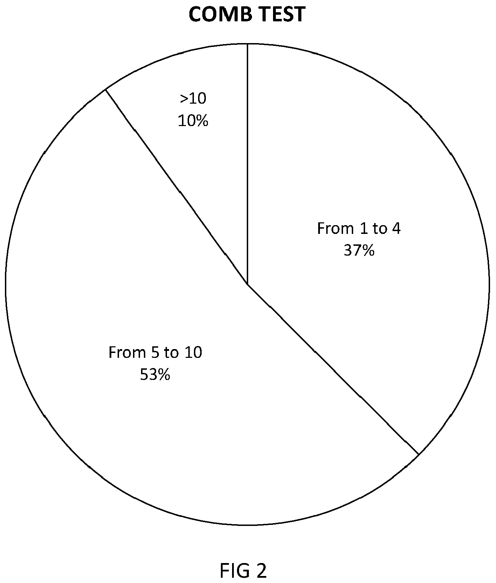

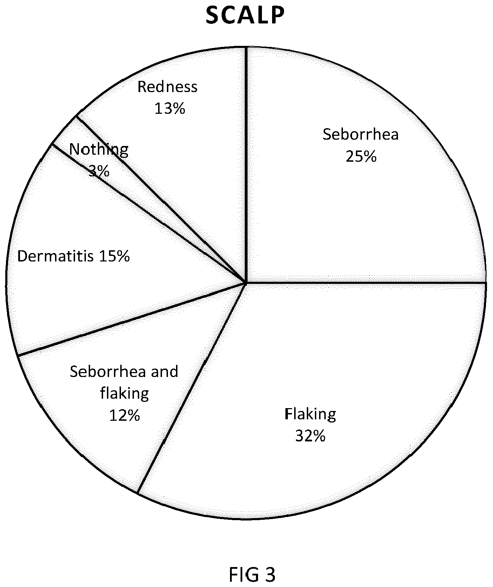

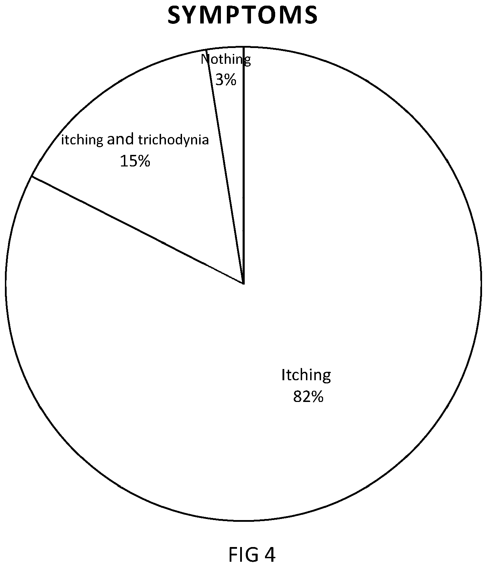

- 25 out of 40 people had a positive pull test (Figure 1)
- 40 out of 40 people had a positive comb test with an average of 6.3 hairs falling out (Figure 2)
- 39 out of 40 people had scalp problems including (Figure 3):
   ∘ 10 out of 40 people had hyper seborrhea
   ∘ 13 out of 40 people presented flaking
   ∘ 5 out of 40 people presented hyper seborrhea combined with flaking
   ∘ 6 out of 40 people had dermatitis
   ∘ 5 out of 40 people presented redness
- 39 out of 40 people presented symptoms such as itching and trichodynia, including (Figure 4):
   ∘ 33 out of 40 people presented itching
   ∘ 6 out of 40 people presented itching and trichodynia
- The 40 testers had an average capillary diameter of 72.67 micrometres with a minimum value of 53.17 micrometres and a maximum value of 105.7 micrometres.

### RESULTS

### At T5, the following results were recorded for the 8 groups:

### Group 1-Formulation 1

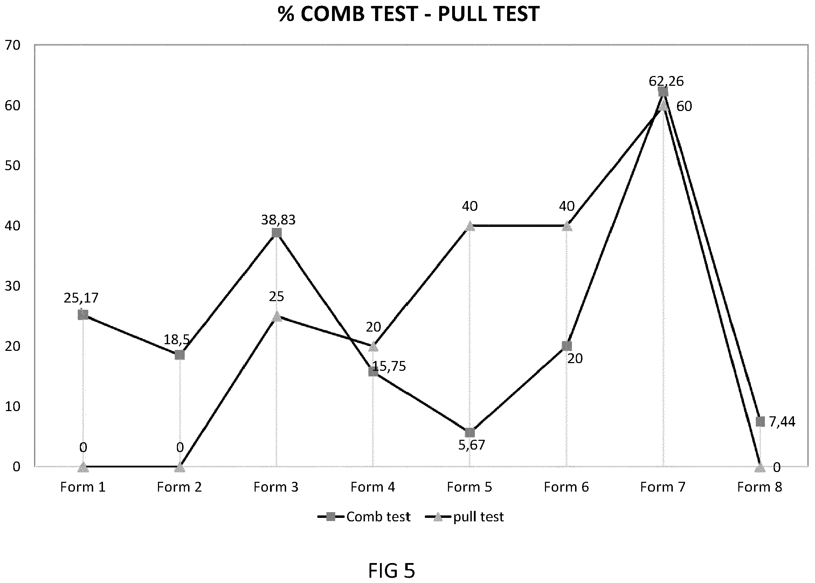

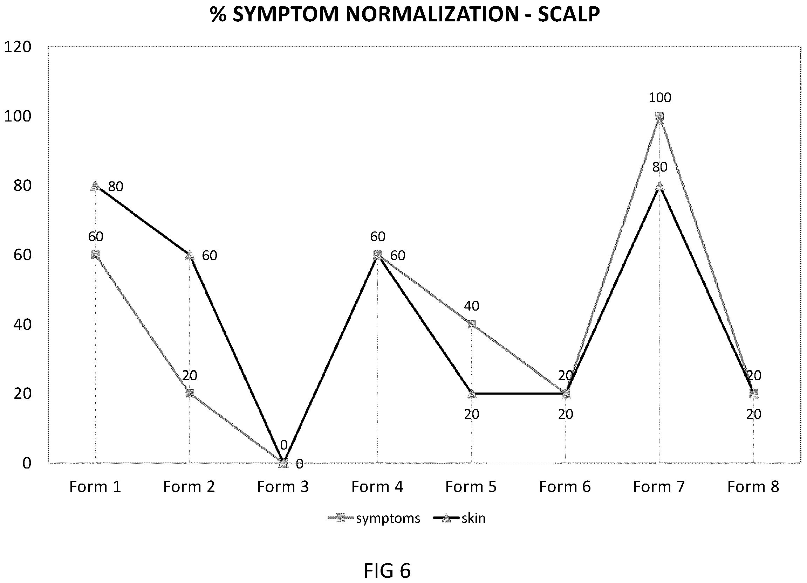

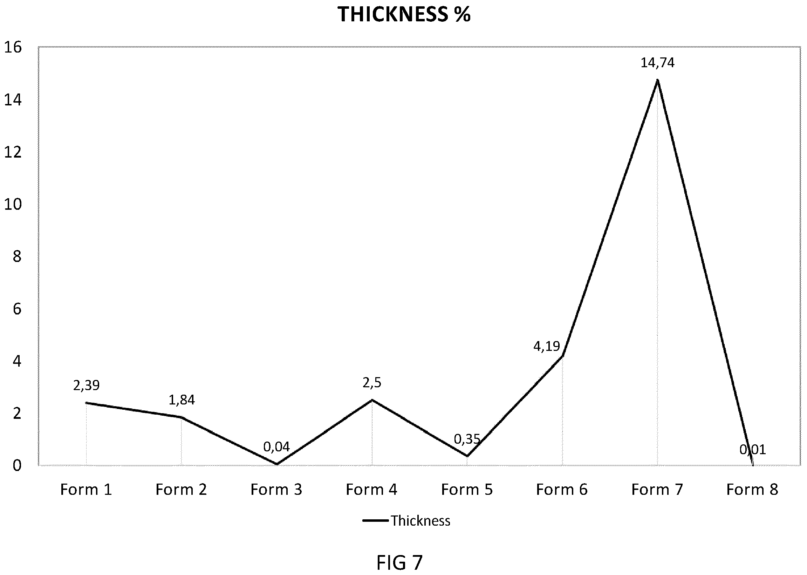

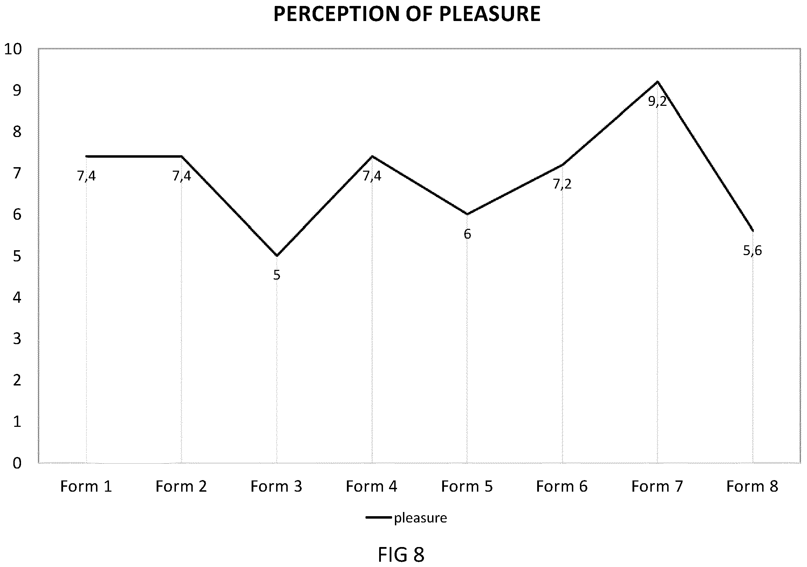

Betaine 2% (632302) + Niacinamide 1% + 26% alcohol + water for the remaining percentage.
- 0 out of 5 people (0%) had a negative pull test (Figure 5)
- 0 out of 5 people (0%) had negative comb test results with an average hair loss of - 25.17% (Figure 5)
- 4 out of 5 people (80%) had normalization of seborrhea - flaking - dermatitis - redness (Figure 6)
- 3 out of 5 people (60%) experienced a strong decrease or elimination of itching and/or trichodynia (Figure 6)
- The 5 testers had an average thickening of 2.39% (Figure 7)
- The 5 testers had an average of 7.4 as their personal post-treatment aesthetic and well-being perception, on a scale of 0 to 10 where 0 indicates severe skin discomfort or very low aesthetic satisfaction and 10 maximum well-being and excellent aesthetic performance (Figure 8).

### GROUP 2 - Formulation 2

Hydrolized Wheat Protein 1% + 26% alcohol + water for the remaining percentage.
- 0 out of 5 people (0%) had a negative pull test (Figure 5)
- 0 out of 5 people (0%) had negative comb test results with an average hair loss of - 18.50% (Figure 5)
- 3 out of 5 people (60%) had normalization of seborrhea - flaking - dermatitis - redness (Figure 6)
- 1 in 5 people (20 %) experienced elimination of itching and/or trichodynia (Figure 6)
- The 5 testers had an average thickening of 1.84% (Figure 7)
- The 5 testers had an average of 7.4 as their personal post-treatment aesthetic and well-being perception, on a scale of 0 to 10 where 0 indicates severe skin discomfort or very low aesthetic satisfaction and 10 maximum well-being and excellent aesthetic performance (Figure 8).

### GROUP 3 - Formulation 3

Caffeine 0.5% + 26% alcohol + water for the remaining percentage.
- 1 in 5 people (20%) had a negative pull test (Figure 5)
- 0 out of 5 people (0%) had negative comb test results with an average hair loss of - 38.83% (Figure 5)
- 0 out of 5 people (0%) had normalization of seborrhea - flaking - dermatitis - redness (Figure 6)
- 0 out of 5 people (0%) experienced a strong decrease or elimination of itching and/or trichodynia (Figure 6)
- The 5 testers had an average thickening of 0.04% (Figure 7)
- The 5 testers had an average of 5 as their personal perception of aesthetics and wellbeing post-treatment, on a scale of 0 to 10 where 0 indicates severe skin discomfort or very low aesthetic enjoyment and 10 maximum wellbeing and excellent aesthetic performance (Figure 8).

### GROUP 4 - Formulation 4

Panthenol 0.5% + 26% alcohol+ water for the remaining percentage.
- 1 in 5 people (20%) had a negative pull test (Figure 5)
- 0 out of 5 people (0%) had negative comb test results with an average hair loss of - 15.75% (Figure 5)
- 3 out of 5 people (60%) had normalization of seborrhea - flaking - dermatitis - redness (Figure 6)
- 3 out of 5 people (60%) experienced a strong decrease or elimination of itching and/or trichodynia (Figure 6)
- The 5 testers had an average thickening of 2.50% (Figure 7)
- The 5 testers had an average of 7.4 as their personal aesthetic and well-being perception post-treatment, on a scale of 0 to 10 where 0 indicates severe skin discomfort or very low aesthetic satisfaction and 10 maximum well-being and excellent aesthetic performance (Figure 8).

### GROUP 5 - Formulation 5

Cucurbita Pepo Fruits/Seed Extract 0.1% + 26% alcohol + water for the remaining percentage.
- 2 out of 5 people (40%) had a negative pull test (Figure 5)
- 0 out of 5 people (0%) had negative comb test results with an average hair loss of -5.67% (Figure 5)
- 1 in 5 people (20%) had normalization of seborrhea - flaking - dermatitis - redness (Figure 6)
- 2 out of 5 people (40%) experienced a strong decrease or elimination of itching and/or trichodynia (Figure 6)
- The 5 testers had an average thickening of 0.35% (Figure 7)
- The 5 testers had an average of 6 as their personal aesthetic and well-being perception post-treatment, on a scale of 0 to 10 where 0 indicates severe skin discomfort or very low aesthetic satisfaction and 10 maximum well-being and excellent aesthetic performance (Figure 8).

### GROUP 6 -Formulation 6

Pisum Sativum Seed Extract 0.1% + 26% alcohol + water for the remaining percentage.
- 2 out of 5 people (40%) had a negative pull test (Figure 5)
- 0 out of 5 people (0%) had negative comb test results with an average hair loss of -20% (Figure 5)
- 1 in 5 people (20%) had normalization of seborrhea - flaking - dermatitis - redness (Figure 6)
- 1 in 5 people (20%) experienced a strong decrease or elimination of itching and/or trichodynia (Figure 6)
- The 5 testers had an average thickening of 4.19% (Figure 7)
- The 5 testers had an average of 7.2 as their personal aesthetic and well-being perception post-treatment, on a scale of 0 to 10 where 0 indicates severe skin discomfort or very low aesthetic satisfaction and 10 maximum well-being and excellent aesthetic performance (Figure 8).

### GROUP 7 - Formulation 7

The complete formulation of the composition according to the present invention: Betaine 2% (632302) + Niacinamide 1% +Hydrolized Wheat Protein 1% + Caffeine 0.5% + Panthenol 0.5% + Pisum Sativum Seed Extract 0.1% + Eucalyptus Globulus Leaf extract 0.2% + Cucurbita Pepo Fruit and/or Seed Extract 0.1% + Betula Alba Leaf Extract 0.05% + 26% alcohol + water for the remaining percentage
- 3 out of 5 people (60%) had a negative pull test (Figure 5)
- 0 out of 5 people (0%) had negative comb test results with an average hair loss of - 62.26% (Figure 5)
- 4 out of 5 people (80%) had normalization of seborrhea - flaking - dermatitis - redness (Figure 6)
- 5 out of 5 people (100%) experienced a strong decrease or elimination of itching and/or trichodynia (Figure 6)
- The 5 testers had an average thickening of 14.74% (Figure 7)
- The 5 testers had an average of 9.2 as their personal post-treatment aesthetic and well-being perception, on a scale of 0 to 10 where 0 indicates severe skin discomfort or very low aesthetic satisfaction and 10 maximum well-being and excellent aesthetic performance (Figure 8).

### GROUP 8 - Formulation 8

Placebo: 26% alcohol + water for the remaining percentage (PLACEBO)
- 0 out of 5 people (0%) had a negative pull test (Figure 5)
- 0 out of 5 people (0%) had negative comb test results with an average hair loss of -7.44% (Figure 5)
- 1 in 5 people (20%) had normalization of seborrhea - flaking - dermatitis - redness (Figure 6)
- 1 in 5 people (20%) experienced a strong decrease or elimination of itching and/or trichodynia (Figure 6)
- The 5 testers had an average thickening of 0.01% (Figure 7). The 5 testers had an average of 5.6 as an aesthetic perception and personal well-being post-treatment, on a scale of 0 to 10 where 0 indicates severe skin discomfort or very low aesthetic satisfaction and 10 maximum well-being and excellent aesthetic performance (Figure 8).

### RESULTS

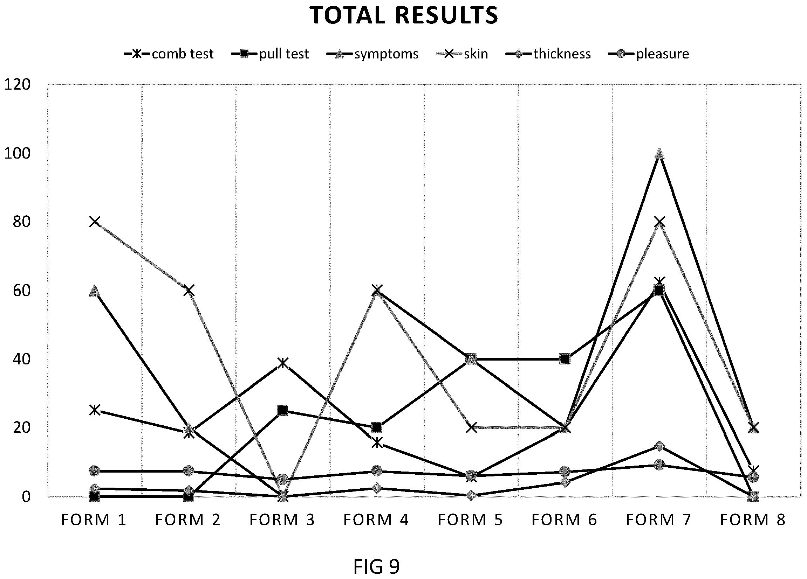

As can be seen in Figure 9, where the results recorded at T5 compared to T0 are shown:
- Group 1, which used a formulation containing only Betaine + Niacinamide, distinguished itself by good results in the following parameters under investigation: normalization of symptoms (60 %) and scalp (80%) and personal satisfaction with the results (7.4).
- Group 2, which used a formulation containing only Hydrolyzed Wheat Protein, stood out in terms of personal satisfaction with the results (7.4).
- Group 3, which used a formulation containing only Caffeine, distinguished itself by good results in decreasing hair loss (-38.83%)
- Group 4, which used a formulation containing only panthenol, performed well in the following parameters under investigation: normalization of symptoms (60%) and scalp (60%) and personal satisfaction with the results (7.4).
- Group 5 which used a formulation containing only *Cucurbita Pepo* did not stand out in any parameter compared to the others.
- Group 6, which used a formulation containing only *Pisum Sativum,* performed well in the following test parameters: pull test negativity (40%), hair shaft thickening (+4.19%) and personal liking of the results (7.2).
- Group 7 that used the complete formulation according to the present invention showed excellent and superior results on all the parameters under study: 60% had negative pull test, there was an average decrease in hair loss of -62.26%, there was normalization of the scalp in 80% and reduction/elimination of symptoms in 100% of those tested. There was an average thickening of +14.74% with an overall rating of 9.2.
- The group 8 that used the placebo formulation did not stand out in any parameter compared to the others.

From the results obtained, it is clear and evident that the synergistic action of the main active ingredients constituting the claimed hair care composition leads to trichological results superior to those achieved by the action of its individual ingredients.

In particular:
at scalp level, the treatment used topically has ensured a cutaneous correction of scalp alterations that are often the cause or concomitant cause of thinning and often associated with annoying and incessant itching, soreness and concomitant scratching wounds.

The synergic action of the complex consisting of betaine, niacinamide, cucurbita pepo, eucalyptus and betula, has been shown to boost skin normalization in cases of redness, desquamation, hyper seborrhea, itching and trichodynia. The normalization promoted by the 5 reported ingredients, together with hydrolyzed wheat proteins, speed up the hydrolipidic rebalancing of the skin, and amplify the result.

Hair growth occurs during the anagen phase, and is promoted by caffeine in synergy with panthenol and *Pisum sativum.* The latter in particular also has an anti-hair loss effect. The study carried out showed that the combination of these ingredients helps to achieve the best results in a short time.

Finally, hydrolyzed wheat proteins associated with *Pisum sativum* have been shown to amplify the thickness of the hair shaft in relation to individual actions.

The hair care composition thus conceived is susceptible to numerous modifications and variations all within the inventive concept described and claimed.

## Claims

1. A hair care composition **characterised in that** it comprises *Pisum sativum* seed extracts as anti-hair loss agents and densifiers for hair growth in synergistic association with betaine, niacinamide, *Cucurbita pepo* seed and/or fruits extracts, eucalyptus extract and birch leaf extracts for use in the treatment of skin alterations of the scalp and for use in the promotion of hair shaft regrowth.

2. The hair care composition according to the preceding claim, **characterised in that** it further comprises hydrolysed wheat proteins in association with said betaine and niacinamide for use in restoring a correct hydrolipidic balance of the skin of the scalp.

3. The hair care composition according to any preceding claim, **characterised in that** it further comprises caffeine and panthenol in synergistic association with *Pisum sativum* seed extracts for use in the treatment of hair loss.

4. The hair care composition according to claim 1, **characterised in that** the amount of *Pisum sativum* extracts present ranges from 0.5 to 2% of the total weight, the amount of betaine present ranges from 0.5% to 3% of the total weight, the amount of niacinamide present ranges from 0.5% to 2% of the total weight, the amount of *Cucurbita pepo* seed extracts present ranges from 0.5 to 2% of the total weight, the amount of eucalyptus extract ranges from 0.5% to 3% of the total weight and the amount of birch leaf extracts ranges from 0.05% to 0.20% of the total weight.

5. The hair care composition according to any preceding claim, **characterised in that** it further comprises propanediol and glycerine as humectant agents, heptyl glucoside as a surfactant agent, tetrasodium glutamate diacetate as a plant-based chelating agent, cetrimonium chloride as an antistatic agent and citric acid as a pH adjusting agent.

6. The hair care composition according to any preceding claim, **characterised in that** it has the following formulation:
| | % of total weight |
|---|---|
| Aqua | 64.2845 |
| Denatured alcohol | 26.190 |
| Propanediol | 2.75 |
| Betaine | 2.00 |
| Glycerine | 1.00 |
| Niacinamide | 1.00 |
| Hydrolysed wheat proteins | 1.00 |
| Caffeine | 0.50 |
| Panthenol | 0.50 |
| Heptyl glucoside | 0.20 |
| *Eucalyptus Globulus* extract | 0.20 |
| Tetrasodium glutamate diacetate | 0.10 |
| *Cucurbita pepo* seed extracts | 0.10 |
| *Pisum Sativum* seed extracts | 0.10 |
| Birch leaf extracts | 0.050 |
| Cetrimonium chloride | 0.025 |
| Citric acid | 0.0005 |

7. The hair care composition according to any preceding claim, **characterised in that** it is in a form suitable for topical application on the skin of the scalp, in particular in the form of shampoos, conditioners, gels, creams, masks and lotions.

8. The hair care composition according to claims 1 and 3 **characterised by** the fact that said extracts in said composition are extracted with a hydro-glycol solvent, in particular propanediol.
